# EUROPEAN PATENT SPECIFICATION

(11) **EP 0 993 625 B1**
(45) Date of publication and mention of the grant of the patent: **28.06.2006**
(21) Application number: 98934288.6
(22) Date of filing: 02.07.1998
(51) Int. Cl.: G03B 21/32

(54) **A SCENT DELIVERY SYSTEM**
DUFTFREIGABESYSTEM
SYSTEME DE LIBERATION DE PARFUMS

(30) Priority: 03.07.1997 US 887622; 12.06.1998 US 97243
(43) Date of publication of application: 19.04.2000
(73) Proprietor: Manne, Joseph, New York, NY 10009 (US)
(72) Inventor: Manne, Joseph, New York, NY 10009 (US)
(74) Representative: Portal, Gérard
(86) International application number: PCT/US1998/013986
(87) International publication number: WO 1999/001793

(56) References cited:
- US-A- 3 628 829
- US-A- 4 310 307
- US-A- 5 109 839
- US-A- 5 610 674

## Description

### BACKGROUND OF THE INVENTION

### 1. Field of Invention

This invention relates to a scent delivery system which provides scented air directly to a user's nose and allows for rapid change at the user's nose of the scented air. The scent delivery system can be combinea with audio and video components to provide a multimedia entertainment system. The scent delivery system can be made portable to allow the user to use the scent delivery system in any location.

### 2. Prior Art

Scent delivery systems which generate scented air are known for use in altering the environment, acting as an alarm, or for use in conjunction with moving images. One problem with the prior art systems is that they are designed to deliver scented air to a large space, such as a room. This means that they need a relatively large amount of scent; cannot react rapidly to changes; and have problems with creating a uniform mixture of scent and air in the space.

US-A-5,610,674 and US-A-3,628,829 show prior art systems in which individual fragrance dispensers are integrated to an individual chair and are foreseen stationary.

Another problem with these prior art systems is that they are so large that they are not portable. It has recently been recognized that scented air can be used to control behavior. For example, appetite and smoking are two cravings which can be controlled with scented air. The need for a smoke or food can occur anywhere, however, the prior art scent delivery systems were not portable.

There is a need for a scent delivery system which allows for rapid changes in the scented air; uses a minimum amount of scent; and allows for use by an individual without offending others in the immediate vicinity of the user of the system. There is also a need for a portable scent delivery system which can be worn by the user and employed anywhere.

### SUMMARY OF THE INVENTION

A scent delivery system has now been discovered which allows for rapid changes in the scented air, uses minimum amount of scent, can be used by an individual without offending others in the immediate vicinity, and which can be portable. The scent delivery system of the present invention can be used in conjunction with audio and/or video components, e.g. a personal computer or television set, to provide a multimedia entertainment system.

The scent delivery system of the present invention employs two main components, a scented air generator and a nasal interface. The scented air generator provides scented air to the nasal interface by means of a conduit. The nasal interface is positioned in close proximity to a user's nose so as to provide scented air directed to the user's nose and allows for a rapid exhaust of the scented air from the user's nose.

Preferably, the scented air generator is contained in a case which houses a number of scent containers. Each container contains a porous pad saturated with a different liquid phase scent. A blower fan is used to force air through the scent container, thereby producing scented air. The scented air is directed to the user's nose by tubing which connects to the nasal interface and exhausts the scented air from under the user's nose. The scented air generator, however, can also employ a single scent container in conjunction with the fan. Additionally, the scented air generator can be one or more aerosol cans of scented air under pressure which drives scented air to the user's nose.

The fan can be replaced with any means that creates an air gradient or pumps air. For example, a canister of compressed air positioned upstream of the scent container or a vacuum positioned at the exhaust of the nasal interface can be used to move the scented air through the system.

The nasal interface provides scented air from the conduit to the nose. Such interfaces include masks that cover the nose, masks that cover both the nose and mouth, or a tubing arrangement which vents scented air directly to the user's nose. The tubing arrangement can be holes in the conduit, a Tee, or a wishbone which is positioned directly below or not more than about 0.25 inches (0.5 cm) into the nasal cavity of the user. The tubing arrangement can be worn by the user about the user's face or can be an arrangement where the user places his or her face in close proximity to their nose. In all cases, the nasal interface must deliver scented air to within close proximity of the user's nose.

A portable scent delivery system of the present invention preferably comprises the features defined in independent claim 1.

Preferably, a scent scrubber is connected to the exhaust tubing of the nasal interface to remove the scent from the air and prevent the scent from escaping from the system. The scent scrubber is preferably housed in the case. This prevents the scented air from offending those who are in the immediate vicinity of the user.

Valves can be used with the system at the inlet, the outlet, or both, of the scent container to prevent the escape of scent during times of non-use. These valves can be manual or electrical. Preferably, the valves are graduated so as to vary the amount of scent released into the air.

Preferably, a microprocessor is housed in the case and controls the fan and the inlet and/or outlet valves of the scent containers. The microprocessor can be programmed so as to operate at certain times or to provide a certain concentration of scent to the air.

Preferably, a biofeedback system is employed with the scent system of the present invention. Suitable biofeedback systems include heart rate monitors, skin galvanometer monitors, and respiratory rate monitors. These biofeedback systems monitor the user and, through the microprocessor, can allow the system to operate as needed by the user. For example, if the heart rate monitor indicates that the user's heart rate has increased, then the microprocessor can activate the scent delivery system to release a calming scent so as to decrease the user's heart rate.

The scent delivery system of the present invention can be employed with electronically reproduced pictures or audio sounds to provide the user with a scent or scents that correspond to a scene being viewed or heard by the user. Because of the ability of the system to rapidly change scented air, the system can keep us the audio and/or video display.

The system of the present invention can operate in conjunction with a wide range of audio and video media, to include movie theater projectors, television and VCR players, radio, computer programs, (including games, CD ROM images and movies), books (and other text displaying devices), and for use with aromatherapy systems, perfume point of sale in conjunction with video, and perfume formulation systems.

To coordinate the scented air with the video images, a mechanism is employed to electronically count the number of NTSC (National Television Standards Committee) signal frames (or other comparable analog video signal conventions) of an incoming video signal. By utilizing characteristic features of the analog signal train, the individual frames can be counted. This enables the synchronization of the video signal with the delivery of scents to the user.

In order to employ the scent delivery system of the present invention with video and/or audio signals, the microprocessor must be programmed to allow for the coordination of the scent to the audio/video presentation.

### BRIEF DESCRIPTION OF THE DRAWINGS

These and other aspects of the present invention may be more fully understood by reference to one or more of the following drawings:
FIG. 1 illustrates a scent delivery system with biofeedback;
FIG. 2 illustrates a scent delivery system with audio and video media;
FIG. 3 illustrates a block diagram of the scent delivery system;
FIG. 4 illustrates the preferred fan;
FIG. 5 illustrates the scent container with manual valves;
FIG. 6 illustrates an electric valve;
FIG. 7 illustrates an automatic valve;
FIGS. 8 and 9 illustrate a Tee tubing nasal interface;
FIG. 10 illustrates a facial mask nasal interface;
FIG. 11 illustrates a nasal mask nasal interface;
FIG. 12 illustrates a wishbone tubing nasal interface;
FIG. 13 illustrates a nasal interface with a short exhaust;
FIG. 14 illustrates another nasal interface; and
FIG. 15 illustrates a simplified version of the present invention.

### DETAILED DESCRIPTION OF THE INVENTION

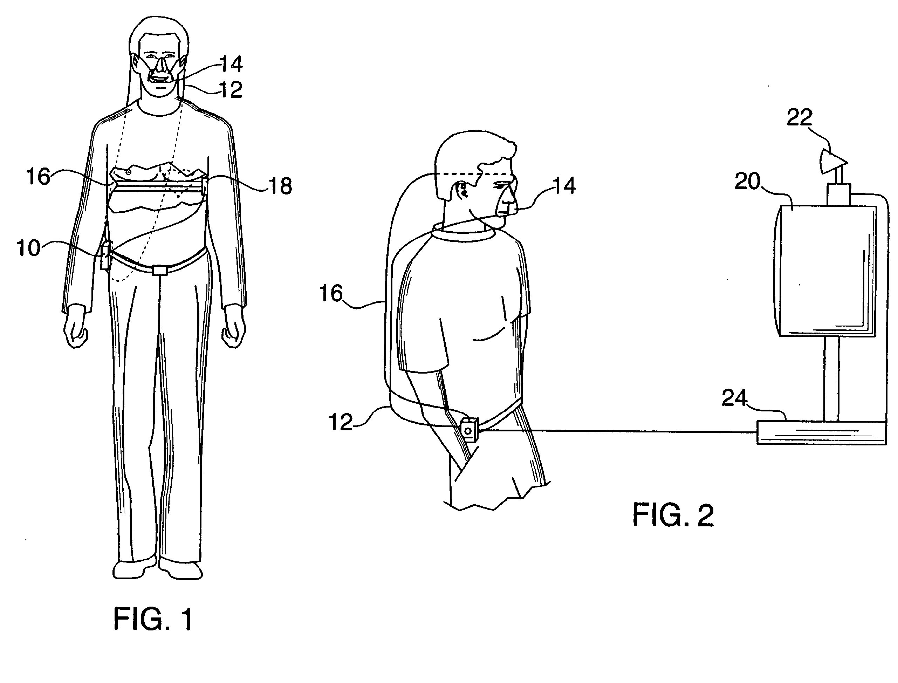

Fig. 1 illustrates a scent delivery system of the present invention wherein compact case 10 houses a scented air generator which is suspended from a user's belt and connected by conduit 12 to nasal interface 14. Conduit 16 exhausts the scented air from nasal interface 14 back to compact case 10 which houses a scent scrubber. Biofeedback system 18 is connected to the scented air generator housed in case 10 to allow the scent delivery system to respond to the user's condition. The scent delivery system shown in Fig. 1 is portable. A microprocessor is housed in case 10 to interact with biofeedback system 18 and control delivery of scented air to the user.

Fig. 2 illustrates a scent delivery system of the present invention interfaced with video display devices 20 and audio device 22, e.g. a computer or a television set. Devices 20 and 22 are electrically connected to a free standing microprocessor 24 which controls the microprocessor in case 10 to coordinate the delivery of scented air to the user with the video images and audio sounds communicated by devices 20 and 22. A biofeedback system can be employed with the multimedia devices 20 and 22 by connecting the biofeedback system to microprocessor 24 so as to coordinate scented air and/or the video and/or audio signals to the condition of the user as understood by the biofeedback system. Control for the system in Fig. 2 comes from free standing external microprocessor 24. Microprocessor 24 includes an A/D converter that can be interfaced with either device 20 or 22 or both. If the signals from devices 20 and 22 are digital, there is no need for the A/D converter or the A/D converter is by-passed. The electronic signal which is used to generate devices 20 and 22 are accessed by the free standing microprocessor 24. For example, microprocessor 24 is capable of interpreting the video signal such as the current frame of the movie. Then, using preprogrammed information, the free standing microprocessor 24 determines the scent or combination of scents that the viewer should be exposed to so that the scented air correspsonds to the video image currently being displayed. Obviously, microprocessor 24 and the microprocessor used in case 10 to control the operation of the scented air generator can be combined into one microprocessor. However, for purposes of manufacture, it may be less expensive to employ two separate microprocessors.

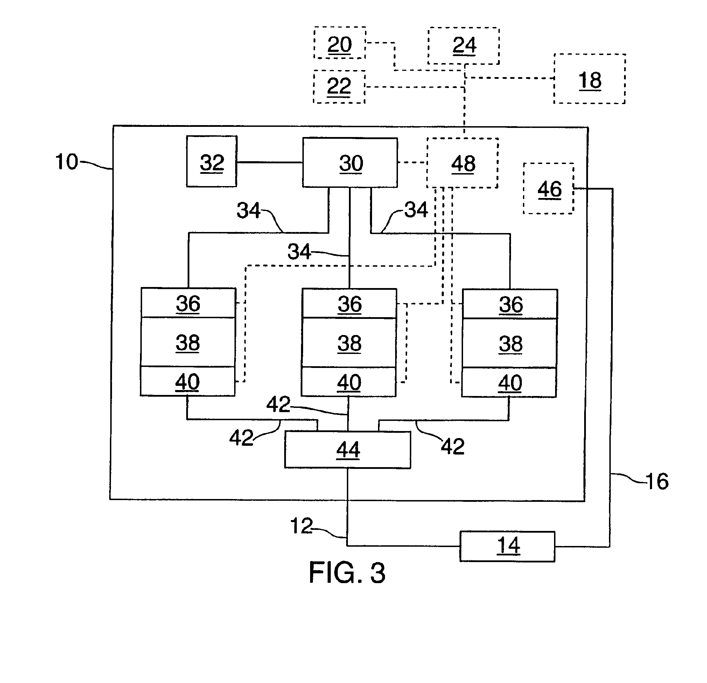

Fig. 3 illustrates a block diagram of the major components of the scented air generator of the present invention. Case 10 is a rectangular box which can be made from a variety of materials including different metals and plastics. The dimensions of the box are preferably small, more preferably, 10cm x 15cm x 6cm. Case 10 contains a blower fan 30. Fan 30 is powered by a 12 volt rechargeable battery pack 32. The fan 30 delivers its output to tubing 34. Tubing 34 directs the fan output into the scent inlet valve 36. Inlet valve 36 directs the air into a scent container 38. The scented air exits scent container 38 by virtue of an open
outlet valve 40. Then, the scented air is delivered through outlet tubing 42 to a packed bed mixer 44.

The packed bed mixer 44 directs the input from all the scent containers 38 into one outlet. This outlet leads to nasal interface tubing 12 which directs the scented air to nasal interface 14 located at the user's nose. The outflow or exhaust from nasal interface 14 passes through the nasal exhaust tubing 16 and is directed into the optional scent scrubber 46 which is also contained within case 10. Optional microprocessor 48 controls fan 30 and inlet and outlet valves 36 and 40. Microprocessor 48 can receive input from optional biofeedback system 18, as well as external microprocessor 24. Alternatively, where microprocessors 48 and 24 are combined into one microprocessor, then video device 20 and audio device 22 provide direct input to microprocessor 48, as shown.

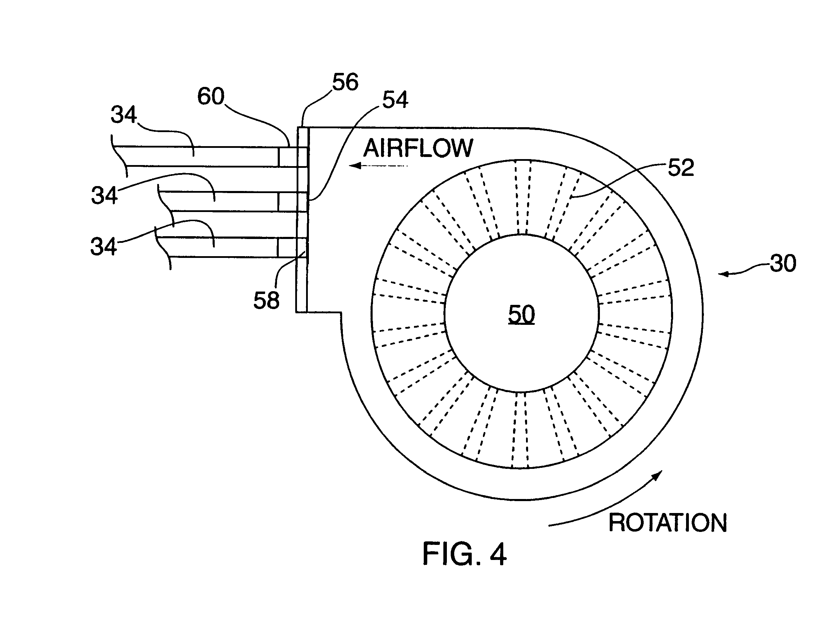

Fig. 4 illustrates the preferred fan 30. Fan 30, in the preferred embodiment, has a squirrel cage configuration. Fan 30 is driven by a brushless DC motor. It has a voltage range of about 10 to about 14 volts. The average current through the motor is about 0.18 amps. The average speed of the motor is about 2600 +/- 200. The diameter of the blower housing is about 7.5 cm. The inlet diameter of the fan is about 4.75 cm. The dimensions of the outlet of the fan is about 3.5 by 2.5 cm. Air enters through inlet 50 and is pushed by blades 52 out outlet 54 as shown.

Fan 30 and its connection with the inlet tubing 34. Overlying the outlet is outlet port plate 56 and outlet ports 58. The dimensions of outlet port plate 56 which overlies the blower outlet has the dimensions about 4.0 cm by 3.0 cm. The outlet ports 58 each have a diameter of about 0.8 cm. Port 58 consists of a 1.0 cm hole drilled into outlet port plate 56. The hole contains plastic flanged barb 60 with an o.d. of 1.0 cm and an i.d of 0.8 cm. The length of barb 60 is about 3.5 cm. In the preferred configuration, there are 6 outlet ports 58 organized in two rows of three. However, this preferred embodiment does not imply that other configurations of outlet ports 58 cannot be used.

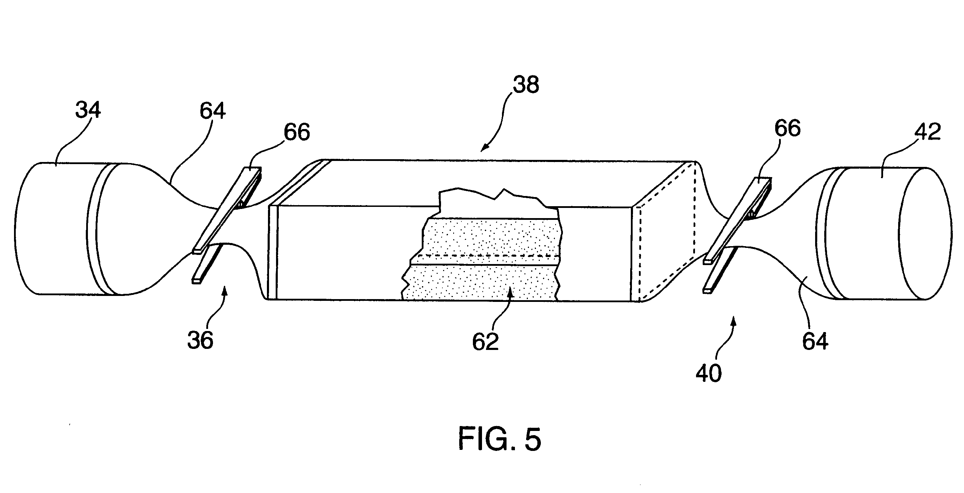

Scent container 38 consists of small rectangular chambers with porous reservoir 62 as shown in Fig. 5. Porous reservoir 62 covers the entire floor of scent container 38. In the preferred embodiment, the dimensions of scent container 38 are: 0.7 cm x 0.7 cm x 7 cm. Porous reservoir 62 is comprised of a porous substance which has sufficient porosity to hold an adequate amount of scent.

Porous reservoir 62 allows the present invention to be portable without an unpredictable dispersion of the liquid throughout scent container 38 as well as into inlet 34 and outlet tubing 42.

In the preferred embodiment, the porous reservoir 62 is an adsorbent material comprised of compressed fibrous strands. The thickness of porous reservoir 62 in the preferred embodiment is about 0.3 mm. One preferred material would be the same type as used in ink pads for use in conjunction with rubber stamps.

Porous reservoir 62 is saturated with the desired liquid scent. In this context, the term "saturation" is meant to refer to the state where all the air-filled spaces in porous reservoir 62 have been replaced with liquid scent. Furthermore it refers to the state where the addition of any more liquid scent would lead to it simply being excluded from the solid and accumulate outside the porous solid. The exact capacity of the solid can be calculated on the basis of porosity data for the solid.

In the preferred embodiment, inlet valve 36 and outlet valve 40 control the flow of air into and out of scent container 38. In the preferred form, inlet valve 36 and outlet valve 40 comprise elastomeric conduit 64 with persistent memory which is externally compressed by clamp 66. Thus, each inlet/outlet is pinched shut.
One preferred elastomer is rubber because of its ability to retain shape memory even after a large number of compressions.

The conduits used in the inlet and outlet valves have an internal diameter of about 1.0 cm and an external diameter of about 1.3 cm.

The external compression which closes the conduit can be performed in a variety of ways. A spring clamp, as shown, is an example of a clamp which would be used. Other types of manual clamping devices and valves can be used, such as a ball valve. Valves 36 and 40 can be manually, electrically, or pneumatically actuated. The valves can be on/off and, more preferably, proportional flow type valves.

Proportional flow type valves allow for variability of the amount of air flow in and out of scent container 38. A racheting type mechanism can be employed with a pinch type clamp to allow for different flows of air into and out of scent container 38.

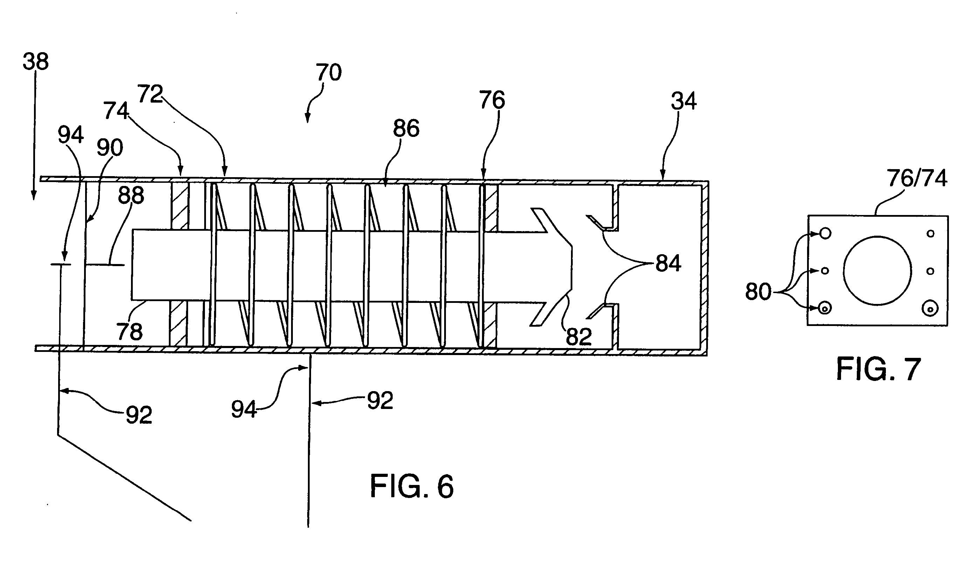

Figs. 6 and 7 show an automated valve 70 which can be used in an alternate embodiment for the pinch valve described in Fig. 5.
Valve 70 can be made out of any solid material including plastic, metal or composites. The outer body of the valve is hollow cylinder 72 with an o.d of 1.5 cm and i.d of 1.2 cm at its ends. Inside the cylinder are two shaft supports labelled 74 and 76, and support valve stem 78. Each is 0.5 cm thick. Each is located 1.5 cm from the nearest end. Their internal diameters are 0.25 cm.

Shaft supports 74 and 76 have multiple perforations 80, each 0.1 cm in diameter, shown in Fig. 7, to allow the passage of compressed air through valve 70. Valve stem 78 travels through the center of shaft supports 74 and 76. Stem 78 has tip 82 which fits into valve seat 84. Stem spring 86 is mounted around stem 78. Spring 86 holds stem 78 firmly against valve seat 84. Spring 86 is held on the left side by shaft support 74 and on the right side by shaft support 76.

Shaft support 76 is affixed to stem 78, and is a thin annular extension of stem 78 which is 0.2 cm thick and has a diameter of 1 cm, thus leaving 0.1 cm clearance to the inside wall of cylinder 72. Stem spring 86 is made such that its uncompressed length is about 25% longer than the distance from shaft support 74 to shaft support 76 when tip 82 sits firmly in valve seat 84.

Under conditions of non-use, stem spring 86 holds stem tip 82 firmly against valve seat 84. Valve seat 84 is machined from the end of hollow cylinder 72. In this position, compressed air cannot flow through it.

One end of cylindrical sleeve 72 is connected to scent container 38 while the other end is connected to inlet tubing 34 which delivers compressed air from fan 30. The direction of compressed air flow is from inlet tubing 34 through valve seat 84 on the right through the body of the valve through perforations 80 in shaft supports 74 and 76 which are shown.

The end of stem 78 opposite the end which sits in valve seat 84 has dynamic alloy wire 88 soldered to it, such as the Flexino^{l}® alloy wire. The other end of wire 88 is attached to a non-conducting grommet which is, in turn, glued or fixed in another way to end 90 of valve 70. Alloy wire 88 is electrically actuated by conducting wire 92, via electrical contact 94, which travels through a hole in the end of sleeve 72 and attaches to the alloy wire near its connection to the non-conductive grommet.

The other end of conducting wire 92 is attached to the body of valve 70 which, in the preferred embodiment, is made from a conductive material so that a circuit is made. The current goes through wire 92 through alloy wire 88 into valve stem 78 to cylinder 72 via its contact with stem spring 86.

Alloy wire 88 has the property that, when it carries an electrical current, it contracts and thus exerts a force. In the preferred embodiment, a 0.2 mm (0.006 inch) diameter alloy wire 88 is used. When it is activated with 400mA DC current, it contracts and exerts a force capable of lifting 330 grams. This force opposes the force of stem spring 86. Thus, it pulls spring loaded stem 78 off valve seat 84 which corresponds to approximately 4% change in alloy wire 88 length. This opens the valve and allows compressed air to pass through. When the current is stopped, alloy wire 88 relaxes and spring 86 pushes stem 70 against valve seat 84 which closes valve 70. The operation of valve 70 is controlled by microprocessor 48 in a conventional manner.

Scent does not travel in the conduits in a homogeneous manner
no matter whether the gas flow is laminar or turbulent. Therefore, a packed bed mixer 44 in the exit flow stream is preferably implemented to guarantee full mixing of the scent. This packed bed mixer is to provide a homogeneous concentration of the scent in the air, whether there is a single scent container or multiple scent containers.

The packed bed mixer is downstream from the scent containers as shown in Fig. 3. All the outlet tubes from the individual scent containers feed into the packed bed mixer. The packed bed mixer is chosen because it is well suited to thoroughly mix gas streams especially when the streams are in laminar flow. A high velocity turbulent gas flow can also be used, however, laminar flow gas streams are preferred. Thus, the packed bed mixer is used in order to guarantee a fully mixed gas stream delivered to the user. Packed bed mixers are conventional and are used in the present invention in a conventional manner.

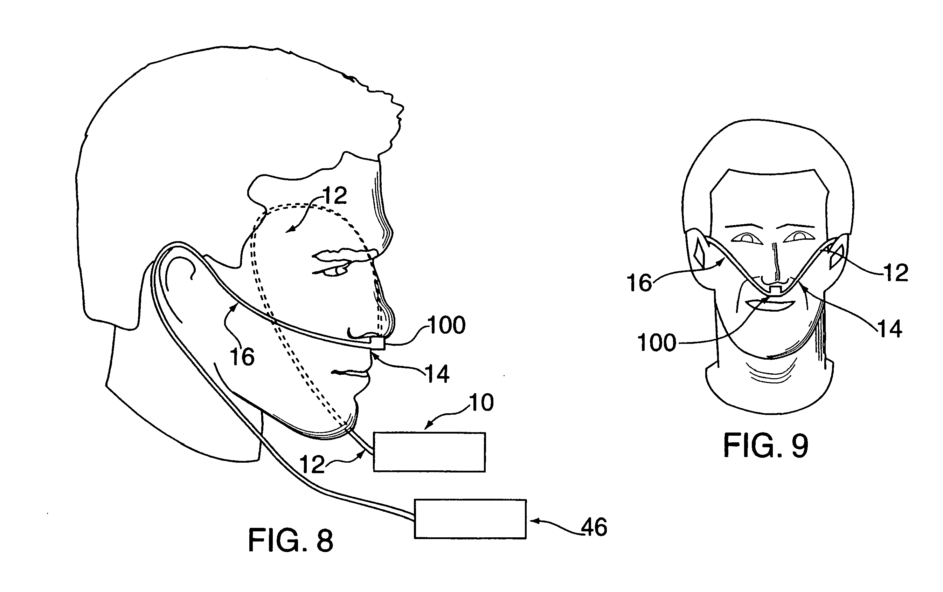

Various embodiments of nasal interface 14 will now be described. Figs. 8 and 9 show nasal interface 14 as Tee 100. Figs. 8 and 9 illustrate the way in which it fits on the user's head. The delivery of the scent to the user is via tubing which wraps around the user's head and passes underneath the user's nose. Tubing 12 carries scented air to Tee 100 which is located underneath the user's nose. Tubing 16 leads away from Tee 100 and acts as nasal exhaust.

Exhaust tubing 16 leads into a scent scrubber 46 which is a box with a charcoal filter which removes the fragrance chemicals from the exhausted air. Tee 100 has a 90 degree branch which lies below the two nostrils. This branch is located on the top side of the tubing which is closest to the nostrils and forms a Tee in the tubing.

Calculating the quantity of the gas and scent delivered to the user of this invention is done in a conventional manner.

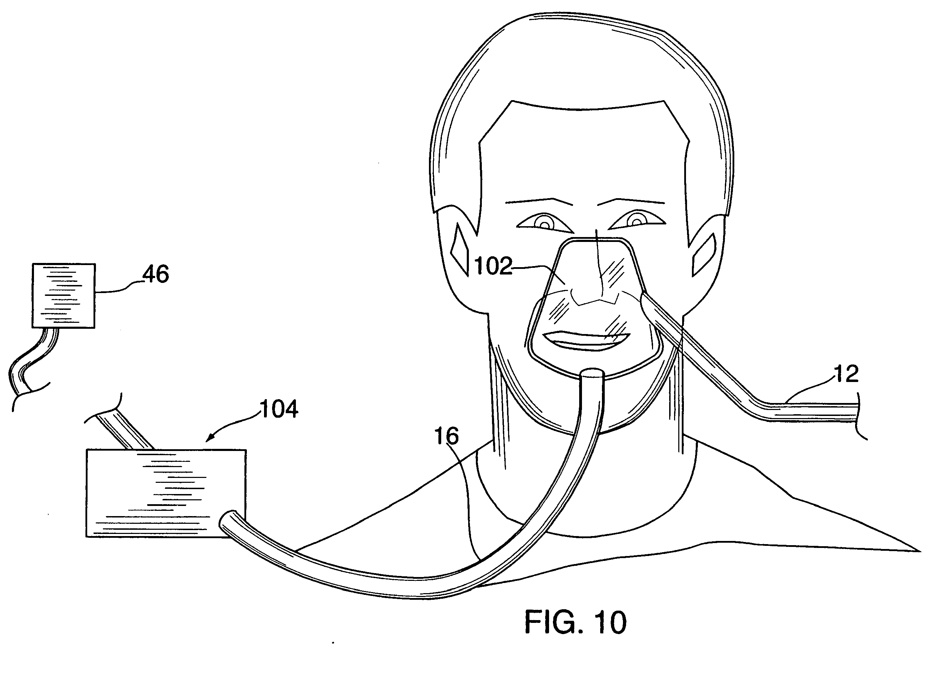

Figs. 10, 11, 12, 13 and 14 show alternate embodiments which can be used as nasal interfaces. As shown in Fig. 10, face mask 102 fits over the user's nose and mouth. The dimensions of the mask is 9.0 cm at the base. The base refers to the base of the triangle below the mouth. The top of the triangle which goes over the bridge of the nose is 4 cm. The sides of the triangle are 14 cm in length. The mask can be made out of any biocompatible substance such as vinyl, or polyethylene. The mask can have a metal crimp over the bridge of the nose to help hold it snugly. Tubing 12 feeds directly into a face mask. The scent is exhausted through tubing 16 with the help of scrubber booster 104 which leads directly into scent scrubber 46.

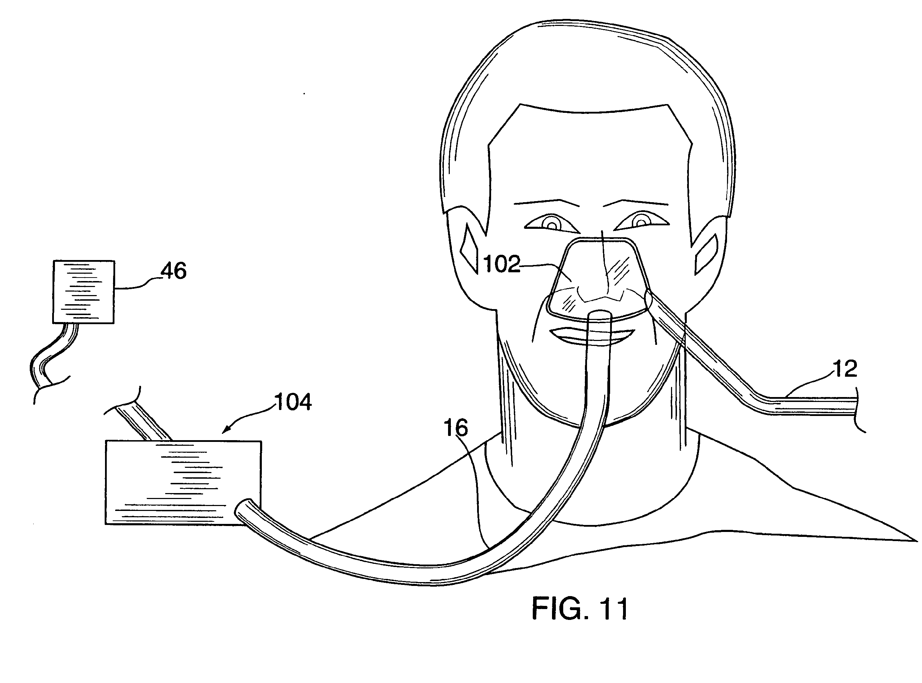

Fig. 11 shows another alternate embodiment. It is nasal mask 106 whose input also comes from tubing 12 and which fits snugly over the user's nose including the nostrils. The dimensions of this mask are that of a triangle whose base is 6 cm and sides are 7cm in length. Within this same mask is a simple outlet which carries the scented air via tubing 16 out to scent scrubber 46. However, the pressure in the mask itself is close to atmospheric pressure, therefore, there is no driving force to push the air through the mask. Therefore, the system has an inline vacuum pump (scrubber booster 104) described in Fig. 10. This pump draws air from the mask and forces it towards scrubber 46.

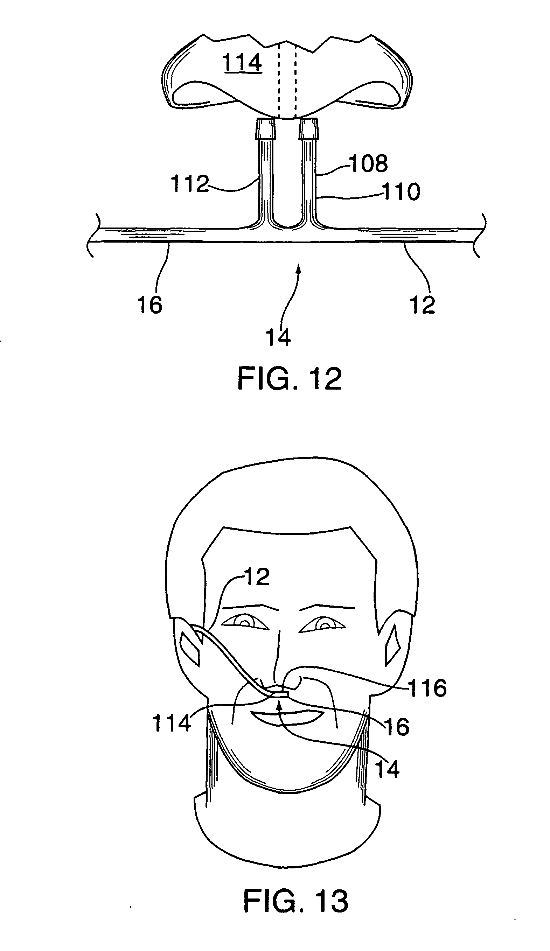

Fig. 12 illustrates nasal interface 14 in a wishbone arrangement of tubing. Wishbone 108 is connected to pipes 12 and 16. Wishbone 108 is made up of branches 110 and 112, each of which extends no more than about 0.5 cm (0.25 inches) into nasal cavity 114 of the user as shown in Fig. 12.

Fig. 13 illustrates nasal interface 14 as two holes 114 and 116 in tubing 12 positioned directly under the user's nose. Exhaust tubing 16 ends directly under the nose of the user. In this embodiment, no scrubber is employed, rather, the scented air exhaust directly to the environment. Exhaust tubing 16 can be opened to allow scented air to move out to the side, or closed so as to force all the scented air out through holes 114 and 116.

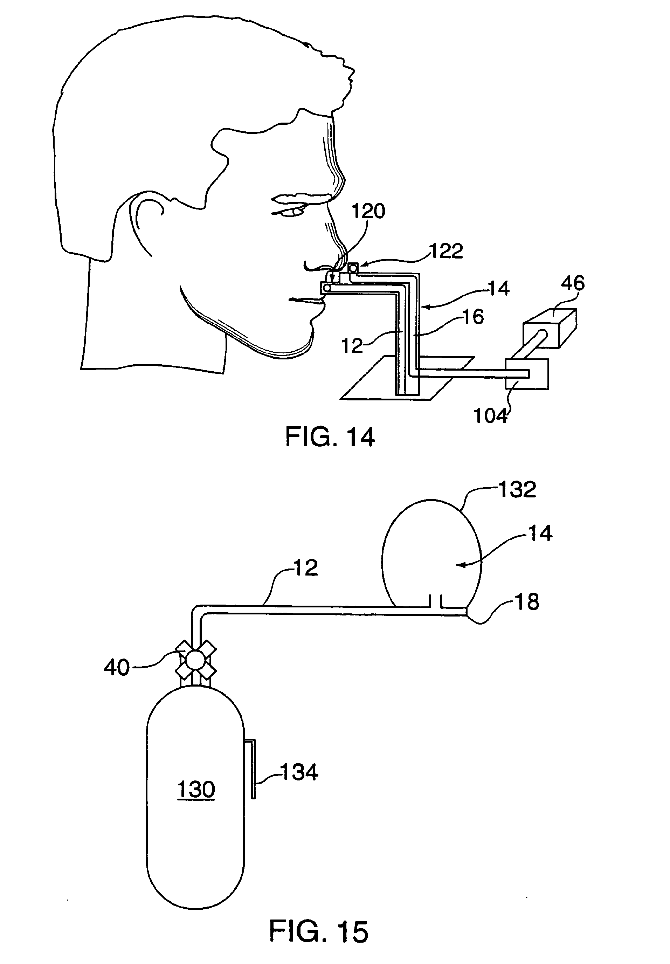

Fig. 14 illustrates nasal interface 14 which sits under a user's nose, output holes 120 are at the end of tubing 12, while exhaust holes 122 are connected to exhaust tubing 16 which in turn is connected to vacuum 104 to draw exhaled scented air from the user's nose and push it to scrubber 46. Nasal interface 14 in Fig. 14 is made of hollow metal tubing through which the flexible tubing 12, 16 passes, as shown.

With the nasal interface as shown in Fig. 14, the user positions nasal interface 14 underneath his nose, rather than wearing it about his face.

As should be appreciated, with a movie theater, each seat is equipped with a nasal interface of one of the types as depicted in Figs. 8-14.

Scrubber 48 receives input from exhaust tubing 16. The scrubber is filled with a filtration material that can remove odors from the air. One common type of filter material is activated carbon. However, there are other filter materials which can also be used. For example, fibrous filters or water soaked porous materials can be used. The air is then exhausted through the scrubber outlet. Such scrubbers are conventional pieces of equipment.

The microprocessor used in the present invention is conventional and programmed in a conventional manner so as to control the fan, the valves and obtain data from the biofeedback system and video/audio systems employed in the present invention. A suitable microprocessor for use in the present invention is a Basic Stamp 2-IC, made by Parallax, Inc. of California, USA.

Biofeedback systems are conventional and are used to obtain data as to the condition of the user..They are especially useful if the scent delivery system of the present invention is intended to have a beneficial alteration on the behavior of the user. Suitable biofeedback systems are heart rate monitors, skin galvanometer monitors, and respiratory rate monitors. These are conventional devices operated in a conventional manner to provide data to the microprocessor which then uses the data to control the scent delivery system of the present invention.

The microprocessor can be preprogrammed to follow a certain pattern or certain amount of dosage to the user. For example, it can be programmed to turn on every four hours for a ½ hour to provide the user with a set dosage of scent in four hour intervals. Likewise, it can be controlled to provide different scents or dosages of scents by controlling the opening width of valves 36 and 40.

A simplified device in accordance with the present invention is shown in Fig. 15 wherein canister 130 of compressed scented air has valve 40 connected to tubing 12, nasal interface 14, and exhaust tubing 18. In order to release the scented air from canister 130, the user opens valve 40 which allows scented air to move through tubing 12 to nasal interface 14. The fact that the air is under pressure in canister 130 provides the force necessary to move the air through tubing 12 and to the user's nose. Nasal interface 14 is Tee 100 as shown in Figs. 8 and 9 with elastic band 132 to hold Tee 100 in place under the user's nose. Canister 130 has chip 134 to fit into a user's belt to allow the device to be portable.

Case 10 in Figs. 1 and 2 can employ one or more canisters with compressed scented air with their respective valves in place of the fan, the scent container and their respective valves.

Additionally, one scent container or one canister can be employed wherein a combination of scents have been premixed so as to avoid the need for the use of the packed bed mixer or to avoid the use of multiple scent containers or multiple canisters.

The use of the packed bed mixer is optional in that it provides for an optimal way to mix multiple scents from individual scent containers.

Additionally, valves 36 and 40 need not be employed in the embodiment shown in Fig. 3. Turning fan 30 on and off will cause the scented air to move through the system. To completely stop the scented air, the user can turn off fan 30 and remove nasal interface 14 from his/her nose. Alternatively, a single valve, either 36 or 40, can be employed in the present invention, preferably valve 40. Valve 40 need not be located next to container 38, but can be located anywhere in the tubing between container 38 and nasal interface 14.

Nasal interface 14 must have an exhaust to allow carbon monoxide/dioxide, which is expelled by the user, to escape. Where nasal interface 14 is a mask, as depicted in Figs. 1, 2, 10 and 11, the exhaust can be the exhaust tube as shown in the drawings, or it can simply be the gaps between the user's face and the mask itself. Typically, such masks are not "airtight" and they allow for the exhale of the user to escape from the user's nose. The nasal interface must allow for the user to exhale and for the exhaled gas to escape from the user's nose in order to avoid asphyxiation of the user.

The term "scent" or "fragrance" has been used herein to refer to a chemical compound or compounds which provide a discernible odor to the user. Pharmaceutical drugs are not intended to be delivered by the system of the present invention because the concentration of the chemical (scent) in the air is too low to allow for effective and/or efficient delivery of a drug to a user through the olfactory senses.

The olfactory organs of humans are located in their nasal cavity. It is known that olfactory stimuli can be achieved through the mouth, however, the efficiency of such a delivery channel is poor. Therefore, the present invention is directed to delivery of the scent primarily by way of the nasal cavity and not the mouth.

It is also known that stimuli of the olfactory organ is obtained with volatile and soluble substances in low concentrations. Thus, the present invention contemplates the use of low concentrations of scent molecules or odor molecules in the air.

A gaseous medium is used to deliver the low concentration of scent to the nasal cavity. The preferred medium is air since it is readily available and probably the safest gas to use. Naturally, other inert gases could be used, such as oxygen or helium, however, from a cost and safety perspective, air is preferred.

The calculation of the concentration of scent in the system can be done using conventional fluid flow equations.

The tubing employed in this invention can be any conventional flexible tubing such as polyethylene.

Each one of valves 36 and 40 can be operated independently of each other to control the amount of scent delivered to the user. This allows for a complex set of scents to be delivered to the user. This allows for the concentration of scent to be controlled and thereby for the scented air to correspond to the visual images provided to the user.

The video and audio systems are connected to the scent delivery system through microprocessor 24 in a conventional manner. For example, the video signal is fed into an analog to digital converter. The voltage input into the A/D converter gets converted into a digital output, which is fed into the microcontroller. The A/D converted and microcontroller are part of microprocessor 24 in Fig. 2. The digital data is in serial form. The data is latched onto the microcontroller using the rising or falling edge of a clock signal line in the typical way that a shift register works. This digital data becomes the input variables for software onboard the microcontroller which then looks up the correct open/close settings for the valves for a given video image. This data is stored in a 32 bit variable called seq(i).

An algorithm is used by the onboard software to find the variable seq(i). Each bit from the 32 bit word "seq(i)" will be used to control the separate valves and the fan. This is accomplished by first using 8 bit serial shift registers. Three pins from the BSII microcontroller are connected to each shift register. Since there are 16 I/O pins on the BSII microcontroller, and three pins are dedicated to each shift register, then 5 shift registers can be controlled by the microcontroller. Since each shift register has eight possible outputs, it is possible to control separate valves with one microcontroller.

## Claims

1. A portable scent delivery system comprising:
a portable case (10) worn by a user ;
a fan (30) for moving scented air through the system, said fan housed in said case;
one or more scent containers (38) housed in said case (10), each of said scent containers (38) having an inlet valve (36) and an outlet valve (40), said inlet valve (36) connected by means of inlet tubing (34) to said fan (30) ;
a mixing bed (44) housed in said case (10), said mixing bed (44) having an inlet (42) connected to said outlet valve (40) of each of said scent containers (38) by means of an outlet tubing (42) and having a single outlet (12) connected to nasal tubing;
a nasal interface (14) for wearing by said user of said scent delivery system at the user's nose so as to deliver scent (12 ; 14) directly to the user's nose and to remove scent (14 ; 16) from the user's nose, said nasal interface (14) having an inlet (12) connected to said nasal tubing and an outlet (16) connected to exhaust tubing; and
an electrical source (32) housed in said case, said electrical source providing electricity to said fan (30) so that said fan (30) can move scented air through said system.

2. The system of claim 1 wherein said nasal interface (14) is a nose mask, a face mask (102), or a T-joint (100).

3. The system of claim 1 or 2 further comprising a scent scrubber (48) connected to the exhaust tubing (16) of the nasal interface to remove the scent from the air and prevent the scent from escaping from the system, said scent scrubber (48) housed in said case (10).

4. The system of any one of claims 1 to 3 further comprising a microprocessor (24) housed in the case (10) for controlling the fan (30) and the inlet (36) and outlet (40) valves of the scent containers (38).

5. The system of any one of claims 1 to 4 further comprising a biofeedback system (18) which is connected to said scent generator so as to provide said scent generator with feedback as to a user and to allow said generator to react to said feedback.

6. The system of any one of claims 1 to 5 wherein the system is connected to a video system (20) for displaying images to a user and said system further comprises a microprocessor (24) to coordinate scented air to said displayed images.

7. The system of any one of claims 1 to 6 wherein the system is connected to an audio system (22) to provide sound to the user and said system further comprises a microprocessor (24) to coordinate scented air to said sound provided to the user.

## Patentansprüche

1. Tragbares Duftabgabesystem, umfassend:
Ein tragbares Gehäuse (10), das von einem Anwender getragen wird;
einen Lüfter (30) zum Bewegen bedufteter Luft durch das System, wobei der Lüfter in dem Gehäuse untergebracht ist;
einen oder mehrere Duftbehälter (38), die in dem Gehäuse (10) untergebracht sind, wobei jeder der Duftbehälter (38) ein Einlaßventil (36) und ein Auslaßventil (40) hat, wobei das Einlaßventil (36) mit einer Einlaßleitung (34) des Lüfters (30) verbunden ist;
ein Mischbett (44), das in dem Gehäuse (10) untergebracht ist, wobei das Mischbett (44) einen Einlaß (42) hat, der mit dem Auslaßventil (40) von jedem der Duftbehälter (38) mittels einer Auslaßleitung (42) verbunden ist und eine einzige Auslaßleitung (12) hat, die mit einer Nasalleitung verbunden ist.
eine Nasalschnittstelle (14) zum Tragen durch den Anwender des Duftabgabesystems an der Nase des Anwenders, um Duft (12; 14) direkt an die Nase des Anwenders abzugeben und Duft (14; 16) von der Nase des Anwenders zu entfernen, wobei die Nasalschnittstelle (14) einen Einlaß (12) hat, verbunden mit der Nasalleitung und einen Auslaß (16), verbunden mit der Auslaßleitung; und
eine Stromquelle (32), die in dem Gehäuse untergebracht ist, wobei die Stromquelle Strom für den Lüfter (30) so liefert, daß der Lüfter (30) beduftete Luft durch das System bewegen kann.

2. Das System nach Anspruch 1, worin die Nasalschnittstelle (14) eine Nasenmaske, eine Gesichtsmaske (102) oder ein T-Stück (100) ist.

3. System nach Anspruch 1 oder 2, weiterhin umfassend einen Duftwäscher (48), der mit der Auslaßleitung (16) der Nasalschnittstelle verbunden ist, um Duft aus der Luft zu entfernen und den Duft am Entweichen aus dem System zu hindern, wobei der Duftwäscher (48) in dem Gehäuse (10) untergebracht ist.

4. System nach einem der Ansprüche 1 bis 3, weiterhin umfassend einen Mikroprozessor (24), der in dem Gehäuse (10) untergebracht ist, um den Lüfter (30) und das Einlaß- und Auslaßventile (40) der Duftbehälter (38) zu kontrollieren.

5. System nach einem der Ansprüche 1 bis 4, weiterhin umfassend ein Biofeedbacksystem (18), welches mit dem Dufterzeuger so verbunden ist, daß der Dufterzeuger mit Rückmeldung versorgt wird, so daß einem Anwender und dem Erzeuger ermöglicht wird, auf die Rückmeldung zu reagieren.

6. System nach einem der Ansprüche 1 bis 5, worin das System mit einem Videosystem (20) verbunden ist, um einen Anwender Bilder anzuzeigen und das System weiterhin einen Mikroprozessor (24) umfaßt, um beduftete Luft mit den angezeigten Bildern zu koordinieren.

7. System nach einem Ansprüche 1 bis 6, worin das System mit einem Audiosystem (22) verbunden wird, um dem Anwendet Ton zu liefern und das System weiterhin einen Mikroprozessor (24) umfaßt, um beduftete Luft mit dem dem Anwender gelieferten Ton zu koordinieren.

## Revendications

1. Système de distribution portable de parfums comprenant :
un boîtier portable (10) porté par un utilisateur ;
un ventilateur (30) destiné à déplacer un air parfumé au travers du système, ledit ventilateur étant logé dans ledit boîtier ;
un ou plusieurs récipients (38) de parfum logés dans ledit boîtier (10), chacun parmi lesdits récipients (38) de parfum ayant un clapet d'entrée (36) et un clapet de sortie (40), ledit clapet d'entrée (36) étant raccordé au moyen d'une tubulure d'entrée (34) audit ventilateur (30) ;
un lit (44) de mélange logé dans ledit boîtier (10), ledit lit (44) de mélange ayant une entrée (42) raccordée audit clapet de sortie (40) de chacun parmi lesdits récipients (38) de parfum au moyen d'une tubulure de sortie (42) et ayant une sortie unique (12) raccordée à une tubulure nasale ;
une interface nasale (14) destinée à être portée par ledit utilisateur dudit système de distribution de parfum au niveau du nez de l'utilisateur afin de distribuer un parfum (12 ; 14) directement dans le nez de l'utilisateur et afin d'éliminer un parfum (14 ; 16) du nez de l'utilisateur, ladite interface nasale (14) ayant une entrée (12) raccordée à ladite tubulure nasale et une sortie (16) raccordée à une tubulure d'évacuation ; et
une source électrique (32) logée dans ledit boîtier, ladite source électrique fournissant de l'électricité audit ventilateur (30) de sorte que ledit ventilateur (30) peut déplacer de l'air parfumé au travers dudit système.

2. Système selon la revendication 1, dans lequel ladite interface nasale (14) est un masque nasal, un masque facial (102), ou un joint en T (100).

3. Système selon la revendication 1 ou 2 comprenant en outre un épurateur (48) de parfum raccordé à la tubulure d'évacuation (16) de l'interface nasale afin d'éliminer le parfum de l'air et empêcher le parfum de s'échapper du système, ledit épurateur (48) de parfum étant logé dans ledit boîtier (10).

4. Système selon l'une quelconque des revendications 1 à 3, comprenant en outre un microprocesseur (24) logé dans le boîtier (10) destiné à commander le ventilateur (30) et les clapets d'entrée (36) et de sortie (40) des récipients (38) de parfum.

5. Système selon l'une quelconque des revendications 1 à 4 comprenant en outre un système (18) de rétroaction biologique qui est raccordé audit générateur de parfum afin de fournir audit générateur de parfum une rétroaction concernant un utilisateur et autoriser ledit générateur à réagir à ladite rétroaction.

6. Système selon l'une quelconque des revendications 1 à 5, dans lequel le système est raccordé à un système vidéo (20) destiné à afficher des images pour un utilisateur et ledit système comprend en outre un microprocesseur (24) afin de coordonner un air parfumé par rapport auxdites images affichées.

7. Système selon l'une quelconque des revendications 1 à 6, dans lequel le système est raccordé à un système audio (22) destiné à fournir du son pour l'utilisateur et ledit système comprend en outre un microprocesseur (24) afin de coordonner un air parfumé audit son fourni à l'utilisateur.
